(19) Europäisches Patentamt
European Patent Office
Office européen des brevets

(11) Veröffentlichungsnummer: **0 327 985 A1**

(12) **EUROPÄISCHE PATENTANMELDUNG**

(21) Anmeldenummer: **89101865.7**

(51) Int. Cl.⁴: **C07C 43/16 , C07C 43/162 , C07C 43/176 , C07C 41/28**

(22) Anmeldetag: **03.02.89**

(30) Priorität: **11.02.88 DE 3804162**

(43) Veröffentlichungstag der Anmeldung: **16.08.89 Patentblatt 89/33**

(84) Benannte Vertragsstaaten: **BE CH DE FR GB LI NL**

(71) Anmelder: **BASF Aktiengesellschaft**
**Carl-Bosch-Strasse 38**
**D-6700 Ludwigshafen(DE)**

(72) Erfinder: **Hoelderich, Wolfgang, Dr.**
**Mannheimer Strasse 18 c**
**D-6710 Frankenthal(DE)**
Erfinder: **Goetz, Norbert, Dr.**
**Schoefferstrasse 25**
**D-6520 Worms 1(DE)**
Erfinder: **Hupfer, Leopold, Dr.**
**Waltershoehe 3**
**D-6701 Friedelsheim(DE)**

(54) **Verfahren zur Herstellung von Vinylethern.**

(57) Die Anmeldung betrifft ein Verfahren zur Herstellung von Vinylethern der Formel

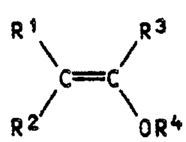
,

in der $R^1$ bis $R^3$ untereinander gleich oder verschieden sind und für Wasserstoff, geradkettige oder verzweigte Alkyl- oder Alkenylreste mit bis zu 12 Kohlenstoffatomen, Cycloalkyl- oder Cycloalkylenreste mit 5 bis 8 Kohlenstoffatomen, Aryl-, Alkylaryl-, Alkenylaryl-, Aralkyl- und Aralkenylreste mit 6 bis 16 Kohlenstoffatomen, halogensubstituierte Arylreste oder für heterocyclische Reste stehen und darüber hinaus die Reste $R^1$ und $R^2$ oder $R^1$ und $R^3$ zusammen mit dem C-Atom, an das sie gebunden sind, ein Cycloalkan, Cycloalken oder einen Heterocyclus bilden können, und $R^4$ für Alkyl- oder Alkylaryl- oder Aralkylreste steht,bei dem man Alkohole aus Acetalen/Ketalen der Formel

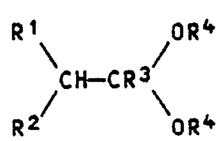
,

in der $R^1$ bis $R^4$ obige Bedeutung haben, in Gegenwart von Phosphaten mit Zeolithstruktur und/oder gefällten Phosphaten der Elemente B, Zr, Ce, Fe und/oder Phosphorsäure bzw. Borsäure auf Trägermaterial und/oder sauren undotierten Metalloxiden als Katalysatoren abspaltet.
Die Umsetzung wird vorzugsweise in der Gasphase durchgeführt.

EP 0 327 985 A1

## Verfahren zur Herstellung von Vinylethern

Die Erfindung betrifft ein Verfahren zur Herstellung von Vinylethern durch Abspaltung von Alkoholen aus Acetalen/Ketalen in Gegenwart von heterogenen Katalysatoren.

Vinylether werden für die Herstellung spezieller Homo- und Copolymerisate eingesetzt, die Anwendung auf den Gebieten der Lack- und Klebstoffherstellung sowie als Hilfsmittel in der Textil- und Lederindustrie finden. Weiterhin dienen Vinylether als wertvolle Zwischenprodukte für organische Synthesen z.B. für Diels-Alder-Reaktionen, für die Herstellung von Glutardialdehyden, γ-Pyran und γ-Picolin sowie Wirkstoffe.

Technisch werden Vinylether nach Reppe aus Acetylen und Alkoholen in Flüssigphase mit Kaliumhydroxid als Katalysator hergestellt. Die Acetylenbasis ist jedoch nicht überall vorhanden, daher ist eine Alternativsynthese für Vinylether wünschenswert.

Ein anderes Verfahren zur Herstellung von Vinylethern ist die Alkoholabspaltung aus Acetalen/Ketalen. Es ist bekannt, daß Alkali-(US 3 705 924), Erdalkali- und Ammoniumphosphate auf Trägermaterial die Umsetzung katalysieren.

Weiterhin ist bekannt, Sulfate wie $NaHSO_4$, Sulfate von Erdalkali- und Schwermetallen oder Alkali-/Erdalkalicarbonate oder CaO (GB 2091-259) auf Trägermaterial als Katalysatoren einzusetzen.

Diese Katalysatoren sind basischer Natur oder die Acidität des verwendeten Trägermaterials ist stark abgeschwächt.

Es wurde nun gefunden, daß man bei der Herstellung von Vinylethern der Formel (I)

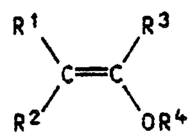

(I),

in der $R^1$ bis $R^3$ untereinander gleich oder verschieden sind und für Wasserstoff, geradkettige oder verzweigte Alkyl- oder Alkenylreste mit bis zu 12 Kohlenstoffatomen, Cycloalkyl- oder Cycloalkylenreste mit 5 bis 8 Kohlenstoffatomen, Aryl-, Alkylaryl-, Alkenylaryl-, Aralkyl- und Aralkenylreste mit 6 bis 16 Kohlenstoffatomen, halogensubstituierte Arylreste oder für heterocyclische Reste stehen und darüber hinaus die Reste $R^1$ und $R^2$ oder $R^1$ und $R^3$ zusammen mit dem C-Atom, an das sie gebunden sind, ein Cycloalkan, Cycloalken oder einen Heterocyclus bilden können, und $R^4$ für Alkyl- oder Alkylaryl- oder Aralkylreste stehen, gute Ergebnisse erzielt, wenn man Alkohole aus Acetalen/Ketalen der Formel (II)

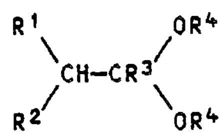

(II),

in der $R^1$ bis $R^4$ obige Bedeutung haben, in Gegenwart von Phosphaten mit Zeolithstruktur und/oder gefällten Phosphaten der Elemente B, Zr, Ce, Fe und/oder Phosphorsäure bzw. Borsäure auf Trägermaterial und/oder sauren undotierten Metalloxiden als Katalysatoren abspaltet.

Als Reste $R^1$ bis $R^3$ kommen unabhängig von $R^4$ Wasserstoff sowie geradkettige oder verzweigte Alkylreste mit 1 bis 12, insbesondere 1 bis 8, vorzugsweise 1 bis 4 Kohlenstoffatomen in Betracht.

Dies bedeutet, daß überraschenderweise und im Gegensatz zur Lehre der Technik auch acide Katalysatoren bzw. Trägermaterialien ohne Dotierung für die Umsetzung sehr gut geeignet sind.

Geeignete Alkyl- oder Alkenylreste sind z.B. Methyl-, Ethyl-, n-Propyl-, i-Propyl-, Propenyl-, i-Propenyl-, n-Butyl-, i-Butyl-, n-Butenyl-, i-Butenyl-, Pentyl-, Pentenyl-, Hexyl-, Hexenyl-, Heptyl-, Heptenyl-, Octyl-, Octenyl-, Nonyl-, Nonenyl-, Decyl-, Decenyl-, Dodecyl- oder Dodecenyl-Reste.

Cycloalkylreste sind z.B. Cyclopentyl-, Cyclohexyl-, Cycloheptyl-, Cyclopentenyl- oder Cyclohexenylreste.

Als aromatische Reste kommen z.B. Phenyl-, Benzyl-, 2-Phenylethyl-, 3-Phenylpropyl-, 2-Phenylpropyl-, 4-Phenylbutyl-, 3-Phenylbutyl, 2-Phenylbutyl- oder 3-Phenylbutenyl-Reste in Betracht, die gegebenenfalls noch durch unter den Reaktionsbedingungen inerte Reste wie Alkylreste oder Halogenatome substituiert sein können.

Heterocyclische bzw. heteroaromatische Reste sind z.B. Tetrahydrofuran-, Dihydrofuran-, Furan-,

Tetrahydrothiophen(Thiophan)-, Dihydrothiophen-, Thiophen-, Pyridin-, Thiopyran-Reste. Diese Reste können noch durch unter den Reaktionsbedingungen inerte Reste wie Alkylreste oder Halogenatome substituiert sein.

Als Rest $R^4$ kommen Alkylreste wie Methyl-, Ethyl-, n-/i-Propyl-, Propenyl-, n-, i-, t-Butyl-, Butenyl-, Octyl-, Octenylreste oder Aralkylreste wie Benzyl-, Phenylethyl-, Phenylpropylreste oder Alkylarylreste wie Toluyl-, Xylolreste in Betracht.

Es kommen Acetale von gesättigten aliphatischen Aldehyden z.B. Acet-, Propion- oder Butyraldehyd, Pentanal, Hexanal und höhere homologe n-Alkanale wie Octanale und Decanale, verzweigte Aldehyde wie Isobutyraldehyd, 2-Methylbutanal, 3-Methylbutanal, 3,3-Dimethylbutanal, 2-Methylpentanal, 2-Ethylhexanal und 2-Methyldecanal zum Einsatz.

Als Ketone für die eingesetzten Ketale kommen beispielsweise die folgenden Verbindungen in Betracht: Methylethylketon, Diethylketon, Methylisopropylketon, Diisopropyl- und Diisobutylketon, Methylisobutylketon, Methoxyaceton, Cyclopentanon, Cyclohexanon, Methylcyclopentanone, Methylcyclohexanone, Cyclohexenon, Acetophenon und substituierte Acetophenone.

Als Katalysatoren für das erfindungsgemäße Verfahren zur Herstellung von Vinylethern werden Phosphate mit Zeolithstruktur und/oder gefällte Phosphate der Elemente B, Zr, Ce, Fe und/oder Phosphorsäure bzw. Borsäure auf Trägermaterial und/oder saure undotierte Metalloxide als Katalysatoren allgemein eingesetzt, insbesondere verwendet man z.B. Aluminiumphosphate bzw. Siliciumaluminiumphosphate bzw. Siliciumeisenaluminumphosphate bzw. Cobaltaluminiumphosphat bzw. Eisenaluminiumphosphat bzw. Boraluminiumphosphate mit Zeolithstruktur, Cerphosphate, Zirkonphosphate, Borphosphate, Eisenphosphate oder deren Gemische.

Als Aluminiumphosphat-Katalysatoren werden insbesondere unter hydrothermalen Bedingungen synthetisierte Aluminiumphosphate eingesetzt. Diese Aluminiumphosphate besitzen Zeolithstruktur.

Als Aluminiumphosphate, die unter hydrothermalen Bedingungen hergestellt sind, kann man z.B. APO-5, APO-9, APO-11, APO-12, APO-14, APO-21, APO-25, APO-31 und APO-33 verwenden. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB) in Wasser homogen mischt, zu dieser Mischung Tetrapropylammoniumhydroxid gibt und danach das Gemisch bei etwa 150 °C 20 bis 60 h unter autogenem Druck in einem Autoklaven umsetzt. Das abfiltrierte $AlPO_4$ wird bei 100 bis 160 °C getrocknet und bei 450 bis 550 °C calciniert.

$AlPO_4$-9 (APO-9) wird ebenfalls aus Orthophosphorsäure und Pseudoboehmit, aber in wäßriger DABCO-Lösung (1,4-Diazabicyclo-(2,2,2)octan) bei etwa 200 °C unter autogenem Druck während 200 bis 400 h synthetisiert.

Die Synthese des $AlPO_4$-21 (APO-21) erfolgt aus Orthophosphorsäure und Pseudobeohmit in wäßriger Pyrrolidon-Lösung bei 150 bis 200 °C unter autogenem Druck während 50 bis 200 h.

Erfindungsgemäß kann man auch Siliciumaluminiumphosphate, z.B. SAPO-5, SAPO-11, SAPO-31 und SAPO-34 verwenden. Die Synthese dieser Verbindungen ist in EP 103 117, US 4 440 871 beschrieben. Diese Siliciumaluminiumphosphate besitzen Zeolithstruktur. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird. Diese Siliciumaluminiumphosphate besitzen Zeolithstruktur.

SAPO-5 beispielsweise wird durch Mischen von $SiO_2$ - suspendiert in wäßriger Tetrapropylammoniumhydroxid-Lösung - mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 168 °C und die Calcination bei 450 bis 550 °C.

Als Siliciumaluminiumphosphate sind auch z.B. ZYT-5, ZYT-6, ZYT-7, ZYT-9, ZYT-11 und ZYT-12 geeignet.

Die so hergestellten Phosphate können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110 °C/16 h getrocknet und bei 500 °C /16 h calciniert.

Man erhält auch vorteilhafte Katalysatoren, wenn das isolierte Phosphat direkt nach der Trocknung verformt wird und erst nach der Verformung einer Calcinierung unterworfen wird. Die hergestellten Phosphate können in reiner Form, ohne Binder, als Stränge oder Tabletten eingesetzt werden, wobei als

Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt das Phosphat, z.B. Siliciumaluminiumphosphat, aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Als Phosphatkatalysatoren kann man auch gefällte Aluminiumphosphate, des B, Fe, Zr, Ce und deren Gemische einsetzen.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 258 bis 650 °C, vorzugsweise 300 bis 500 °C, herstellen.

Wenn bei der erfindungsgemäßen Verwendung der Phosphat-Katalysatoren eventuell eine durch Koksabscheidung bedingte Desaktivierung eintritt, empfiehlt es sich, die Phosphat-Katalysatoren durch Abbrennen der Koksablagerung mit Luft oder mit einem Luft/$N_2$-Gemisch bei 400 bis 550 °C zu regenerieren.

Durch partielle Verkokung (pre-coke) ist es möglich, die Aktivität des Katalysators für ein Selektivitätsoptimum des gewünschten Reaktionsproduktes einzustellen.

Um eine hohe Selektivität, hohen Umsatz sowie lange Standzeiten zu erreichen, kann es vorteilhaft sein, die Phosphat-Katalysatoren zu modifizieren. Eine geeignete Modifizierung der Katalysatoren besteht z.B. darin, daß man das unverformte oder verformte Phosphat mit Metallsalzen durch einen Ionenaustausch oder durch Imprägnierung dotiert. Als Metalle werden Metalle der 3., 4. und 5. Hauptgruppe wie Al, Ga, Ge, Sn, Pb, Ni, Übergangsmetalle der 4. bis 8. Nebengruppe wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppen wie Cu, Ag, Zn, Seltene Erdmetalle wie La, Ce, Pr, Nd, Er, Yb und U eingesetzt.

Zweckmäßigerweise führt man die Dotierung so durch, daß man das verformte Phosphat in einem Steigrohr vorlegt und bei 20 bis 100 °C eine wäßrige oder ammoniakalische Lösung eines Halogenids oder eines Nitrats der voranbeschriebenen Metalle überleitet (Ionenaustausch). Eine weitere Möglichkeit der Metallaufbringung ist gegeben, indem man das Phosphat mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht darin, daß man $Cu(NO_3)_2 \times H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst. Mit dieser Lösung wird der verformte oder unverformte Phosphat eine gewisse Zeit, etwa 30 Minuten, getränkt. Die eventuell überstehende Lösung wird am Rotationsverdampfer von Wasser befreit. Danach wird das getränkte Phosphat bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(CO_3)_2$-Lösung oder ammoniakalische $Pd(CO_3)_2$-Lösung herzustellen und darin das reine pulverförmige Phosphat bei 40 bis 100 °C unter Rühren etwa 24 h aufzuschlämmen. Nach Abfiltrieren, Trocknen bei etwa 150 °C und Calcinierung bei etwa 500 °C kann der so gewonnene Phosphatkatalysator mit oder ohne Bindemittel zu Strängen, Pellets oder Wirbelgut weiterverarbeitet werden.

Eine weitere Möglichkeit der Modifizierung besteht darin, daß man das Phosphat - verformt oder unverformt - einer Behandlung mit Säuren wie Salzsäure, Flußsäure und/oder Wasserdampf unterwirft. Dabei geht man vorteilhaft so vor, daß man das Phosphat vor oder nach seiner Verformung mit Bindemitteln 1 bis 3 Stunden bei Temperaturen von 60 bis 80 °C mit einer 3 bis 25 gew.%igen, insbesondere 12 bis 20 gew.%igen wäßrigen Salzsäure behandelt. Anschließend wird das so behandelte Phosphat mit Wasser gewaschen, getrocknet und bei 400 bis 500 °C calciniert.

Eine besondere Ausführungsform für die Säurebehandlung besteht darin, daß man das Phosphat vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von 0,5 bis 5, vorzugsweise 1 bis 3 Stunden. Nach Isolierung, z.B. durch Abfiltrieren und Auswaschen, des Phosphats wird dieses zweckmäßig bei Temperaturen von 100 bis 160 °C getrocknet und bei Temperaturen von 450 bis 600 °C calciniert. Gemäß einer weiteren bevorzugten Ausführungsform für die Säurebehandlung wird das Phosphat nach einer Verformung mit Bindemittel bei erhöhter Temperatur, zweckmäßig bei Temperaturen von 50 bis 90 °C, insbesondere 60 bis 80 °C, über einen Zeitraum von 0,5 bis 5 Stunden mit 12 bis 20 gew.%iger Salzsäure behandelt. Anschließend wird das

Phosphat ausgewaschen und zweckmäßig bei Temperaturen von 100 bis 160 °C getrocknet und bei Temperaturen von 450 bis 600 °C calciniert. Man kann auch an eine HF-Behandlung eine HCl-Behandlung anschließen.

Geeignete Katalysatoren sind auch die sauer wirkenden undotierten Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, weiterhin Titandioxid, Zirkondioxid, Phosphoroxide, Vanadiumoxide, Nioboxide, Boroxide, Aluminiumoxide, Chromoxide, Eisenoxide, Molybdänoxide, Wolframoxide oder Bims oder Gemische dieser Oxide. Die Behandlung mit Säuren, wie oben beschrieben, ist eine Möglichkeit der Modifizierung.

Auch mit Phosphorsäure oder Borsäure getränkte Katalysatoren können eingesetzt werden. Phosphorsäure oder Borsäure wird auf $SiO_2$-, $Al_2O_3$- oder Bims-Träger oder anderen sauren Metalloxiden als Trägermaterial, z.B. durch Auftränken oder Versprühen aufgebracht. Ein phosphorsäurehaltiger Katalysator kann beispielsweise durch Auftränken von $H_3PO_4$ auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Für das erfindungsgemäße Verfahren werden folgende Reaktionsbedingungen gewählt:

Die Umsetzung führt man vorteilhaft in der Gasphase bei Temperaturen von 100 bis 500 °C, insbesondere von 150 bis 350 °C, bei einem Druck von 0,1 bis 100 bar, insbesondere 0,5 bis 10 bar durch. In der Gasphase wird vorteilhaft eine Katalysatorbelastung von 0,1 bis 20, insbesondere von 1 bis 10 g Ausgangsstoff der Formel II je g Katalysator und Stunde eingehalten (WHSV = g Ausgangsstoff/g Katalysator und Stunde). Die

Reaktion in der Gasphase kann in einem Festbett oder in einem Wirbelbett ausgeführt werden. Es ist auch möglich, die Reaktion in der Flüssigphase (Suspension-, Riesel- oder Sumpffahrweise) bei Temperaturen zwischen 50 und 200 °C durchzuführen. Die Durchführung der Reaktion kann diskontinuierlich, aber vorzugsweise kontinuierlich erfolgen. Besonders vorteilhaft kann verminderter Druck sein.

Schwerflüchtige oder feste Ausgangsstoffe werden in gelöster Form, z.B. in THF-, Toluol- oder Petrolether-Lösung eingesetzt. Generell ist eine Verdünnung des Ausgangsstoffs mit derartigen Lösungsmitteln oder mit Inertgasen wie $N_2$, Ar, $H_2O$-Dampf möglich.

Nach der Umsetzung werden die entstandenen Produkte durch übliche Verfahren, z.B. Destillation aus dem Reaktionsgemisch, isoliert; nichtumgesetzte Ausgangsstoffe werden gegebenenfalls in die Umsetzung zurückgeführt.

Ein besonderer Vorteil ergibt sich, wenn man die gasförmigen Reaktionsprodukte sofort in eine Trennung einbringt und anschließend in ihre Einzelkomponenten zerlegt. Eine derartige Trennung kann z.B. in einer Fraktionierkolonne durchgeführt werden. Eine bevorzugte Ausführungsform ist es, die Reaktionsausträge in einer wäßrigen Hydrogencarbonat-Lösung, z.B. $KHCO_3$ oder $NaHCO_3/Na_2SO_4$, abzukühlen.

Beispiele 1 bis 15

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgekühlt, abgetrennt und charakterisiert. Die quantitative Bestimmung der Reaktionsprodukte und der Ausgangsprodukte erfolgte gaschromatographisch nach bekannter Methode.

Die für das erfindungsgemäße Verfahren eingesetzten Katalysatoren sind:

Katalysator A

$AlPO_4$-5 (APO-5) wird synthetisiert, indem man 200 g 98 %ige Phosphorsäure und 136 g Boehmit in 335 g Wasser löst bzw. suspendiert, hierzu 678 g einer 30 %igen wäßrigen Tetrapropylammoniumhydroxid-Lösung zugibt und diese Mischung in einem Rührautoklaven bei 150 °C in 43 h unter autogenem Druck umsetzt. Nach Abfiltrieren wird das kristalline Material bei 120 °C getrocknet und 500 °C/16 h calciniert. Das so synthetisierte $AlPO_4$-5 enthält 45,5 Gew.% $Al_2O_3$ und 46,5 Gew.% $P_2O_5$. Dieses Material wird mit Pseudo-Boehmit im Massenverhältnis 60 : 40 zu 2-mm-Strängen verformt, abermals bei 120 °C getrocknet

und bei 500° C/16 h calciniert.

Katalysator B

$AlPO_4$-12 (APO-12) wird synthetisiert, indem man 200 g 98 %ige Phosphorsäure und 136 g Boehmit in 400 g Wasser löst bzw. suspendiert, hierzu eine wäßrige Lösung aus 60 g Ethylendiamin und 320 g $H_2O$ zugibt und diese Mischung in einem Rührautoklaven bei 200° C während 24 h unter autogenem Druck umsetzt. Nach Abfiltrieren wird das kristalline Material bei 120° C getrocknet und bei 500° C/16 h calciniert. Das so synthetisierte $AlPO_4$-12 enthält 55,5 Gew.% $P_2O_5$, 39,7 Gew.% $Al_2O_3$. Dieses Material wird mit Verstrangungshilfsmittel zu 3-mm-Strängen verformt, abermals bei 120° C getrocknet und bei 500° C/6 h calciniert.

Katalysator C

Siliciumaluminiumphosphat-5 (SAPO-5) wird hergestellt aus einer Mischung aus 200 g 98 %ige Phosphorsäure, 136 g Boehmit, 60 g Kieselsol (30 %ig), 287 g Tripropylamin und 587 g $H_2O$. Diese Mischung wird bei 150° C während 168 h unter autogenem Druck umgesetzt. Nach Filtration wird das kristalline Produkt bei 120° C getrocknet und bei 500° C/16 h calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, abermals bei 120° C getrocknet und bei 500° C calciniert.

Katalysator D

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100° C getrocknet und bei 350° C calciniert. Katalysator E enthält 8,77 Gew.% B und 28,3 Gew.% P.

Katalysator E

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120° C getrocknet und bei 450° C calciniert. Katalysator F enthält 17,1 Gew.% Ce und 12,7 Gew.% P.

Katalysator F

Im Handel erhältliches Zirkonphosphat (CZP 100®) wird mit Verformungshilfsmitteln zu 2-mm-Strängen verformt, bei 110° C getrocknet und bei 500° C/16 h calciniert.

Katalysator G

$SiO_2$ im Handel erhältlich als D 11-10®.

Katalysator H

100 g $SiO_2$-Stränge (D 11-10) werden mit 280 ml 0,1 n HF und 80 ml $H_2O$ unter Rückfluß 1 h behandelt. Danach wird das Material neutral gewaschen, bei 110° C getrocknet und bei 500° C/5 h calciniert.

Katalysator I

D 10-10 wird mit $H_3BO_3$ imprägniert, getrocknet bei 110 °C und bei 500 °C/5 h calciniert. Der Katalysator I setzt sich zusammen aus 85 % $Al_2O_3$ und 15 % $B_2O_3$.

Katalysator J

Katalysator J wird erhalten, indem man D 10-10 $Al_2O_3$ mit 85 %iger $H_3PO_4$ 30 min behandelt, danach 130 °C/2 h trocknet und bei 540 °C/2 h calciniert. Der P-Gehalt beträgt 4,9 Gew.%.

Katalysator K

$TiO_2$ P 25® wird zu 2-mm-Strängen verformt, bei 110 °C getrocknet und bei 500 °C/16 h calciniert.

Katalysator L

200 g Katalysator G werden mit 600 ml 15 %iger HCl bei 80 °C/1 h behandelt. Danach wird das Material Cl-frei gewaschen, bei 110 °C getrocknet und bei 600 °C/1 h calciniert.

Die Versuchsergebnisse und die Reaktionsbedingungen sind in der folgenden Tabelle zusammengestellt.

Reaktion A) 1,1-Dimethoxyethan ⟶ Methylvinylether + Methanol

Reaktion B) 1,1-Dimethoxy-2-methylpropan ⟶ 1-Methoxy-2-methyl-prop-1-en + Methanol

Reaktion C) 1,1-i-Propoxy-butan ⟶ 1-i-Propoxy-but-1-en + Isopropanol
mit THF 75 : 25 verdünnt

Reaktion D) 1,1-Dimethoxy-octan ⟶ 1-Dimethoxyoct-1-en + Methanol
mit THF 75 : 25 verdünnt

Reaktion E) 1,1-Dimethoxy-2-phenylethan ⟶ 1-Methoxystyrol + Methanol
mit THF 50 : 50 verdünnt

Reaktion F) 1,1-Dimethoxy-2-[p-fluorphenyl]ethan ⟶ 1-Methoxy-p-fluorstyrol + Methanol
mit THF 50 : 50 verdünnt

Reaktion G) Hexyl-dimethoxymethan ⟶ Cyclohexanmethylenmethylether + Methanol
mit THF 50 : 50 verdünnt

Reaktion H Cyclohexanondiethylketal ⟶ Cyclohexenylethylether + Ethanol
mit THF 50 : 50 verdünnt

Verdünnung in Gew.% angegeben.

Tabelle 1

| Beispiel | 1 | 2 | 3 | 4 | 5 | 6 | 7 | 8 | 9 |
|---|---|---|---|---|---|---|---|---|---|
| Reaktion | A) | A) | A) | A) | A) | A) | A) | A) | A) |
| Katalysator | A | C | C | D | E | F | H | I | K |
| Temperatur °C | 350 | 300 | 350 | 300 | 300 | 320 | 300 | 320 | 320 |
| WHSV $h^{-1}$ | 2 | 2 | 3,5 | 2 | 2 | 2 | 2 | 2 | 2 |
| Umsatz % | 90,3 | 100 | 100 | 100 | 95,8 | 87,9 | 100 | 94,7 | 97,3 |
| Selektivität % | 84,7 | 97,3 | 93,8 | 95,4 | 90,3 | 85,2 | 88,7 | 86,9 | 89,5 |

Tabelle 2

| Beispiel | 10 | 11 | 12 | 13 | 14 | 15 | 16 | 17 | 18 |
|---|---|---|---|---|---|---|---|---|---|
| Reaktion | B) | B) | C) | C) | D) | E) | E) | E) | E) |
| Katalysator | C | D | D | E | C | B | C | D | E |
| Temperatur °C | 300 | 300 | 300 | 300 | 300 | 300 | 300 | 300 | 300 |
| WHSV $h^{-1}$ | 2 | 2 | 2 | 2 | 2 | 2 | 2 | 2 | 2 |
| Umsatz % | 100 | 100 | 100 | 98,4 | 100 | 92,7 | 100 | 100 | 100 |
| Selektivität % | 94,1 | 92,7 | 93,5 | 87,6 | 94,0 | 87,7 | 91,8 | 90,5 | 89,3 |

Tabelle 3

| Beispiel | 19 | 20 | 21 | 22 | 23 | 24 | 25 | 26 |
|---|---|---|---|---|---|---|---|---|
| Reaktion | E) | E) | E) | E) | F) | G) | G) | H) |
| Katalysator | E | J | L | K | D | C | D | D |
| Temperatur °C | 300 | 300 | 300 | 300 | 300 | 300 | 300 | 300 |
| WHSV $h^{-1}$ | 2 | 2 | 2 | 2 | 2 | 2 | 2 | 2 |
| Umsatz % | 97,9 | 95,9 | 100 | 98,4 | 100 | 100 | 100 | 100 |
| Selektivität % | 80,6 | 86,1 | 88,2 | 90,2 | 92,3 | 83,2 | 84,3 | 87,9 |

## Ansprüche

1. Verfahren zur Herstellung von Vinylethern der Formel (I)

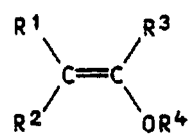

(I),

in der $R^1$ bis $R^3$ untereinander gleich oder verschieden sind und für Wasserstoff, geradkettige oder verzweigte Alkyl- oder Alkenylreste mit bis zu 12 Kohlenstoffatomen, Cycloalkyl- oder Cycloalkylenreste mit 5 bis 8 Kohlenstoffatomen, Aryl-, Alkylaryl-, Alkenylaryl-, Aralkyl- und Aralkenylreste mit 6 bis 16 Kohlenstoffatomen, halogensubstituierte Arylreste oder für heterocyclische Reste stehen und darüber hinaus die Reste $R^1$ und $R^2$ oder $R^1$ und $R^3$ zusammen mit dem C-Atom, an das sie gebunden sind, ein Cycloalkan, Cycloalken oder einen Heterocyclus bilden können, und $R^4$ für Alkyl- oder Alkylaryl- oder Aralkylreste steht, dadurch gekennzeichnet, daß man Alkohole aus Acetalen/Ketalen der Formel (II)

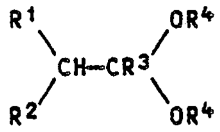

(II),

in der $R^1$ bis $R^4$ obige Bedeutung haben, in Gegenwart von Phosphaten mit Zeolithstruktur und/oder gefällten Phosphaten der Elemente B, Zr, Ce, Fe und/oder Phosphorsäure bzw. Borsäure auf Trägermaterial und/oder sauren undotierten Metalloxiden als Katalysatoren abspaltet.

2. Verfahren nach Anspruch 1, dadurch gekennzeichnet, daß man als Katalysatoren hydrothermal hergestellte Aluminiumphosphate oder Siliciumaluminiumphosphate oder Siliciumeisenaluminiumphosphate oder Eisenaluminiumphosphate oder Boraluminiumphosphate oder Cobaltaluminiumphosphate mit Zeolithstruktur oder deren Gemische verwendet.

3. Verfahren nach den Ansprüchen 1 bis 3, dadurch gekennzeichnet, daß man die Umsetzungen in der Gasphase durchführt.

Europäisches Patentamt

# EUROPÄISCHER RECHERCHENBERICHT

Nummer der Anmeldung

EP 89 10 1865

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-C- 560 354 (CONSORTIUM FÜR ELEKTROCHEMISCHE INDUSTRIE)<br>* Insgesamt * | 1,3 | C 07 C 43/16<br>C 07 C 43/162<br>C 07 C 43/176<br>C 07 C 41/28 |
| X | CHEMICAL ABSTRACTS, Band 24, No. 15, 10. August 1930, Seite 3675,3755, Columbus, Ohio, US; M. CABANAC: "The catalytic decomposition of acetals by metallic oxides", & COMPT. REND. 190, 881-2(1930)<br>* Zusammenfassung * | 1,3 | |
| X | E. MÜLLER et al.: "Methoden der Organischen Chemie", Band VI/3, 1965, 4. Auflage, Teil 3, Seiten 97-102, Georg Thieme Verlag, Stuttgart, DE<br>* Zusammenfassung * | 1,3 | |
| D,A | US-A-3 705 924 (R.A. SMITH)<br>* Tabelle I * | 1-3 | |
| D,A | GB-A-2 091 259 (CIBA-GEIGY)<br>* Ansprüche * | 1-3 | RECHERCHIERTE SACHGEBIETE (Int. Cl.4)<br>C 07 C 41/00<br>C 07 C 43/00 |
| A | EP-A-0 217 089 (DEGUSSA)<br>* Ansprüche * | 1-3 | |
| P,A | EP-A-0 299 286 (BASF)<br>* Ansprüche * | 1-3 | |

Der vorliegende Recherchenbericht wurde für alle Patentansprüche erstellt

| Recherchenort | Abschlußdatum der Recherche | Prüfer |
|---|---|---|
| DEN HAAG | 19-05-1989 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument